# EUROPEAN PATENT APPLICATION

(11) **EP 2 377 543 A2**
(43) Date of publication of application: **19.10.2011**
(21) Application number: 11173627.8
(22) Date of filing: 04.04.2003
(51) Int. Cl.: A61K 36/82, A61P 31/00

(54) **Ophthalmic, pharmaceutical and other healthcare preparations with naturally ocurring plant compounds, extracts and derivatives**

(30) Priority: 04.04.2002 US 117533
(62) Divisional of application: 03726194.8
(71) Applicant: Natural Disinfectant Technologies, Curacao (AN)
(72) Inventor: Christ, F. Richard, Laguna Beach, California 92651 (US); De Bruijn, Chris, 48683 Ahaus (DE); Dziabo, Anthony J., Lake Forest, California 92630 (US); Vigh, Joseph, Placentia, California (US)
(74) Representative: Eke, Philippa Dianne

(57) **Abstract**

A number of discrete, isolated and well-characterized natural plant compounds show anti-microbial activity when used for topical applications in the ophthalmic, skin care, oral care, pharmaceutical, medical device, health care products or similar preparations for topical application. Of particular interest are Green Tea Extract, Oleuropein and Pomegranate Extract. Green Tea Extract, Oleuropein and Pomegranate, either alone or in combination, are extremely effective antimicrobial agents.

## Description

### FIELD OF THE INVENTION

The present invention relates to the use of natural plant compounds, extracts and derivatives alone or in combination or with other chemical antimicrobial agents to preserve ophthalmic, skin care, oral care, pharmaceutical and other healthcare preparations and methods to disinfect soft and rigid gas permeable (RGP) contact lenses.

### BACKGROUND AND DESCRIPTION OF RELATED ART

Ophthalmic, oral care, skin care solutions, emulsions, ointments, gels, creams and many other pharmaceutical and healthcare preparations for topical application (*e.g*., artificial tears, skin creams, mouthwashes, therapeutics, contact lens care products, anti-allergenic, anti-puretics, etc.) must be preserved to prevent biological contamination and degradation. By "preparation for topical application" we mean any cream or solution or other physical form that is applied to the skin, eyes or externally accessible mucous membranes such as preparation inserted into various body orifices. It is now acceptable practice to add chemical preservatives to such preparations to ensure preservation of said preparations. These chemical preservatives (*e.g*., Benzalkonium Chloride, polyhexamethyl biguanide [PHMB], Chlorhexadine, Thimerosol, sorbic acid, *etc*.) are often harsh, synthetic cytotoxic agents, which can irritate and possibly damage sensitive tissues. The same issue applies to any other pharmaceutical and healthcare preparations, which require preservative to prevent biological contamination and degradation.

Currently there is ever increasing interest in "natural" foods and medicines. This may be due to a growing perception on the part of the public that unwanted environmental consequences and unexpected side effects of synthetic chemicals make the use of "natural" alternatives (derived from plant and other natural sources) increasingly attractive. For instance, there is a perception as well as experimental evidence that naturally derived agents have fewer or no adverse side effects. A prior art alternative to the use of chemical disinfecting agents is the use of an extract based on grapefruit in ophthalmic solutions was disclosed by one of the present inventors (De Bruijn International Application PCT/NL97/00092 and Dutch patent NL-1002484). Presumably, the effective agent includes phenolic compounds (*e.g*., bioflavonoids). The parent of the present application demonstrated that natural plant derived substances; such as bioflavonoids, can be employed in contact lens care products as natural disinfecting or preserving agents-either alone or in conjunction with synthetic disinfecting agents. The use of bioflavonoids is desirable because they are natural plant products with antioxidant and even anti-inflammatory properties; however, the majority of bioflavonoids known to date are in the form of complex combinations that are difficult to obtain, manufacture and assay.

In this invention we have discovered that many natural plant compounds, extracts and derivatives have useful antimicrobial properties due to the role that has evolved for such substances in the plant's own natural defense system. These natural plant compounds also have a very different toxicity profile as compared to existing commonly used synthetic chemical antimicrobial compounds. Application of these chemical antimicrobial compounds for preservation or disinfection is a balancing act between maximizing the "cidal" action against contaminating microbes while minimizing the toxicity to the tissue (cells) that the preservation and disinfection are in fact trying to protect from microbial attack. This balancing act is most often accomplished by varying the concentration of the chemical antimicrobial compounds. Optimization of the use of these chemical agents involves determining a minimum concentration sufficient to pass accepted standards of performance (preservation or disinfection) while maintaining acceptable toxicity (which may manifest itself as irritation or other sequellae). Cytotoxicity is a term used to describe the toxicological effects at the cellular level. An antimicrobial compound that would be cytotoxic to the pathogenic organisms, yet not cytotoxic to the tissue and cells of the treatment target would provide a new and beneficial method of preservation and/or disinfection. Benefits of such a preservative and/or disinfection system would be reduced irritation, greater comfort in application, greater compliance (due to the comfort and ease of use) and fewer overall symptoms of discomfort in the use of the product.

This invention has application in the several fields (i.e., ophthalmology, pharmaceuticals, skin care, oral care, hard surface disinfection, OTC (over the counter) Products, etc.). However it would be especially useful in ophthalmic contact lens care products and particularly in contact lens disinfection. Currently available contact lenses are made of hydrogels and other polymers causing them to be soft and hydrophilic so that they can be comfortably worn. Previously, contact lenses were hard plastic (PMMA) and required the contact lens wearing patient to adjust to the uncomfortable sensation of a foreign body in the eye. The advent of soft contact lenses has resulted in an increased adoption of contact lenses by the general population.

Contact lenses are commonly worn on a daily basis and kept in a storage case/solution during the night hours or whenever they are not being worn. During the wear and normal handling of contact lenses, microorganisms as well as biomolecules such as lipids, proteins, etc. can become adhered to the contact lenses and thus transferred to the storage case/solution.

Some of the microorganisms that may be transferred from the eye or fingers to the storage case/solution may multiply therein and may later be pathogenic to the human cornea or other ocular structures upon subsequent lens wear. Although human tears contain natural antimicrobial agents, a pathogen-bearing lens in contact with the cornea of the eye can serve as a reservoir of infection that might overcome the eye's natural defenses. This is especially the case for soft contact lens, as the microorganisms tend to adhere to the lens material. The result of microbial growth-bacterial, protozoan or even fungal-can cause damage to the eye resulting in impaired vision and even blindness. Wearing contact lenses creates an increased exposure to eye infections due to the stress contact lenses place on the cornea and conjunctiva suppressing oxygen conduction and tear flow and/or creating inflammatory/irritating conditions. Therefore, contact lenses should be daily disinfected to eliminate pathogenic organisms

Disinfection agents typically used for other applications such as hard surface disinfection, instrument disinfection, topical skin disinfection, etc. are not necessarily applicable to contact lens care and ophthalmic, pharmaceutical and other healthcare product preservation. These chemical disinfection or antimicrobial agents have usually been designed to destroy all types of cells through an indiscriminate mechanism, whether the cells are target pathogenic microorganisms or corneal epithelial cells in contact with a soft contact lens. The method of action of these chemical agents is unable to discriminate between the target pathenogenic organisms and the tissue that they are intended to protect.

The high concentration used to ensure effectiveness and the chemically aggressive nature of many of these chemical disinfectant agents render them unsuitable for use with contact lenses due to interaction or damage to the lens or irritation to ocular tissue due to residual disinfection agents that become bound to or included within the contact lenses. Commonly used preservative and disinfection agents are compounds such as thimerasol, chlorhexidine, hydrogen peroxide, and benzalkonium chloride. For example, three percent (3%) hydrogen peroxide instilled directly in the eye or a lens soaked in hydrogen peroxide and applied to the eye will result in pain and severe irritation. Only a few compounds at very low concentration levels have been shown to be compatible with soft contact lenses or the tissues of the eye.

Multipurpose solutions (MPS) with chemical disinfection agents, as disclosed in U.S. Patents 4,407,791, 4,525,346, 4,758,595, 4,820,352, 4,836,956, 5,422,073, 5,560,186,5,593,637, and 5,756,045, are widely used for contact lens disinfection.. With the MPS the wearer need only purchase and use a single solution leading to advantages in cost and convenience. The challenge of disinfection without harm to the eye or the lens is particularly acute with these MPS products, however, since all of the various activities, *e.g*., wetting, contaminant dispersion, and disinfection, are required to co-exist in a single solution without antagonistic effects of one component on the activity of another. Furthermore, because the MPS can be instilled directly into the eye, the active antimicrobial component of these solutions must provide the required degree of pathogen reduction while being free of irritating or damaging sequelae to the surface and the anterior segment of the eye or to the contact lens itself. There is no opportunity with an MPS to neutralize or rinse away the disinfecting agent prior to applying the contact lens to the eye.

Generally therefore the art has found it difficult to formulate these MPS solutions to satisfy the following performance criteria. The successful solution must:
1. Show antimicrobial activity to reduce the numbers of common pathogens found on contact lenses to prescribed levels;
2. Formulated at a sufficiently low concentration so as to be nonirritating to the eye without the help of rinsing and/or neutralizing solutions;
3. Be free of toxic metals or compounds and sensitizing agents so that no long term allergic or toxic response is provoked;
4. Not adversely accumulate within or on the lens or adversely alter the wettability or the parameters (*i.e*., size, shape, and optical properties) of the lens or be released in amounts toxic to the eye during lens wear;
5. Show adequate shelf-life (*e.g*., chemical stability);
6. Be compatible with enzymes and other agents used in artificial tears or similar accessories to contact lens wear.

Many of these same criteria apply to successful agents for the disinfection and/or preservation of pharmaceuticals or other healthcare and personal care products-especially those intended for topical application (topical preparations). These agents must show ability to reduce common pathogens found on skin, in the mouth or in other application sites. They must be non-irritating to the tissues at the site of application (particularly critical with applications to the eye or mucus membranes). They must be non-allergenic and free of toxic metals, etc. They must show sufficient stability to prevent growth of contaminating organisms during an adequate shelf life, and they must not be reactive or damaging to active pharmaceutical agents in the topical preparations. Many pharmaceutical and diagnostic preparations that are ingested or injected also require antimicrobial preservation. The naturally occurring preservatives disclosed within are nontoxic and most can be safely ingested.

Disinfectant and preservative tests are most often performed by challenging the preparation with a concentrated inoculum (*e.g*., 10⁵-10⁶ colony forming units (cfu)/ml) of each test organism. Over time samples are taken and plated on a growth medium to estimate the number of live organisms remaining at each time point.

The method for evaluating the effectiveness of a disinfectant for contact lenses generally requires measuring the ability of the agent to reduce the numbers of viable infective organisms during a period of time consistent with the normal period of storage of contact lenses between wearing times. This reduction ofnumbers of organisms is typically reported in terms of the change in the common logarithm (base 10 logarithm) of the microbial population as a result of exposure to the antimicrobial or disinfecting agent. For example, if the agent has effected a reduction in the concentration of a particular organisms in a challenge solution from 10⁶ colony forming units (cfu) per milliliter (ml) to 10² cfu/ml within six hours of exposure then the change, or "log reduction", of the organism as a result of exposure to the agent would be 4.0 (logs). In other words, the number of viable organisms have been reduced to one ten-thousandth of the original level.

In procedures for verifying the effectiveness of ophthalmic antimicrobial agents generally recognized guidelines call for the use of *Candida albicans* (a yeast), *Fusarium solani* or *Aspergillus niger* (both molds), *Pseudomonas aeruginosa* (a Gram-negative bacterium), *Staphylococcus aureus* (a Gram-positive bacterium), *Serratia marcescens* (a Gram-negative bacterium) and *Escherichia coli* (a bacterium common in the human gastrointestinal tract).

In the case ophthalmic solutions and preparations various agents are added to enhance compatibility with the eye. To avoid stinging or irritation it is important that the solution possess a tonicity and pH within the physiological range, *e.g*., 200-350 mOsmole for tonicity and 6.5-8.5 for pH. To this end, various buffering and osmotic agents are often added. The simplest osmotic agent is sodium chloride since this is a major solute in human tears. In addition propylene glycol, lactalose, trehalose, sorbitol, mannitol or other osmotic agents may also be added to replace some or all of the sodium chloride. Also, various buffer systems such as citrate, phosphate (appropriate mixtures of Na₂HPO₄, NaH₂PO₄, and KH₂PO₄ and K₂HPO₄), borate (boric acid, sodium borate, potassium tetraborate, potassium metaborate and mixtures thereof), bicarbonate, and tromethamine (TRIS) and other appropriate nitrogen-containing buffers (such as ACES, BES, BICINE, BIS-Tris, BIS-Tris Propane, HEPES, HEPPS, imidazole, MES, MOPS, PIPES, TAPS, TES, Tricine) can be used to ensure a physiologic pH between about pH 6.0 and 9.0.

Various viscosity building agents such as polyethylene glycol, surfactants, polyvinylpyrrolidone, polyvinyl alcohol, carboxymethyl cellulose, hyaluronic acid, polysaccarides and similar materials may be added to adjust the lubricity, i.e., the "body" and "feel" of the solution. Surface-active agents may be added to ensure proper wetting and/or cleaning. Sequestering agents such as ethylenediaminetetraacetic acid (EDTA), phosphonates, citrate, gluconate and tartarate are also common additives for preservative, disinfecting or cleaning solutions.

As is well known to one of skill in the art similar factors are involved in formulating preparations for application to sites apart from the eye. Most of the ophthalmic formulation factors mentioned above are applicable to preparations designed for application to mucus membranes. For application within the mouth/throat taste and possible toxicity upon ingestion must also be considered. Some oral preparations are intended to resist rapid dilution and washing away by saliva. In such cases, thickening polymers may be especially important. Preparations for dermal application maybe formulated as creams, which are often oil in water emulsions, as gels, which are generally thickened with water soluble or miscible polymers, or as ointments, which are mostly hydrophobic ingredients like waxes and mineral oils.

To date, the significant challenge in the development of ophthalmic and contact lens solutions, particularly the MPS solutions, has been to find disinfection agents with sufficient antimicrobial activity that are not at the same time damaging to the contact lens or irritating to the eye. Due to the complex requirements to keep soft, hydrogel contact lenses clean, free ofpathogen microbes, and comfortable to wear without damaging or changing the lens polymer or dimensional parameters and without any harm or side effects to the human eye, only very few compounds or systems have been qualified as suitable ophthalmic or contact lens solutions. In the parent application to the present application it was disclosed that a particular quaternary ammonium salt (BenzylDimethyl-[2-[2-[(p-1,1,3,3 Tetramethyl butyl) phenoxy)ethoxy]ethyl] ammonium chloride) was unexpectedly effective and non-irritating when used in preservative and cleaning solutions. It was also shown that this material could be combined with certain natural disinfectant agents of plant origin. The present application discloses that certain of natural disinfectant agents are particularly effective and non-toxic even when used without quaternary ammonium salts or other "traditional" disinfecting agents.

Preservation of ophthalmic solutions and pharmaceutical and healthcare topical preparations is similar to disinfection in that a preservative is added to a product to deal with any microbial contamination that might occur during storage or use of the product. Use of any product that is contaminated with microorganisms increases the risk that infection may occur. For instance with artificial tears-products often prescribed to patients suffering from "dry eye" or other tear deficiencies-the product may be used by the patient multiple times over the course of a single day. Although a chemical preservative insures that the artificial tear solution is free of microbial contamination, the patient doses his/her eye with the chemical preservative as well as the artificial tear solution. In a compromised eye (*e.g*., low tear production) the preservative may be responsible for acute and chronic irritation. A preservative chronically used in healthy tissue can cause an acute or latent irritation.

Reference to the biological literature will reveal a plethora of recent studies on irritation caused by a common quaternary ammonium disinfectant BAK. The interested reader should examine these and related articles: Debbasch C, Brignole F, Pisella PJ, Warnet JM, Rat P, Baudouin C: Quaternary ammoniums and other preservatives? Contribution in oxidative stress and apoptosis on Chang conjunctival cells. Invest Opthalmol Vis Sci ; 42: 642-652(2001); Burgalassi S, Chetoni P, Monti D, Saettone MF: Cytotoxicity of potential ocular permeation enhancers evaluated on rabbit and human corneal epithelial cell lines. Toxicol Lett 122: 101-108 (2001); Baudouin C, Pisella PJ, Fillacier K, Goldschild M, Becquet F, De Saint Jean M, Bechetoille A: Ocular surface inflammatory changes induced by topical antiglaucoma drugs: human and animal studies. Ophthalmology; 106: 556-563 (1999); De Saint Jean M, Brignole F, Bringuier AF, Bauchet A, Felmann G, Baudouin C: Effects of benzalkonium chloride on growth and survival of Chang conjunctival cells. Invest Ophthalmol; 40:619-630 (1999); Becquet F, Goldschild M, Moldovan MS, Ettaiche M, Gastaud P, Baudouin C: Histopathological effects of topical ophthalmic preservatives on rat corneoconjunctival surface. Curr Eye Res; 17:419-425 (1998); Saarinen-Savolainen P, Jarvinen T, Araki-Sasaki K, Watanabe H, Urtti A: Evaluation of cytotoxicity of various ophthalmic drugs, eye drop excipients and cyclodextrins in an immortalised human corneal epithalial cell line. Pharm Res; 15: 1275-1280 (1998); Fabreguette A, Zhi Hua S, Lasne F, Damour A: Evaluation of the cytotoxicity of antiseptics used in current practise on cultured of fibroblasts and keratinocytes. Pathol Biol (Paris); 42: 888-892 (1994) Vaughan JS, Porter DA: A new in vitro method for assessing the potential toxicity of soft contact lens care solutions. CLAO J; 19:54-57 (1993); Tripathi BJ, Tripathi RC, Kolli SP: Cytotoxicity of ophthalmic preservatives on human corneal epithelium. Lens Eye Toxic Res; 9: 361-375 (1992); and Withrow TJ, Brown NT, Hitchins VM, Strickland AG: Cytotoxicity and mutagenicity of ophthalmic solution preservatives and UVA radiation in L5178Y cells. Photochem Photobiol; 50:385-389 (1989).

### SUMMARY OF THE INVENTION

It has been discovered that a variety of discrete, isolated and well-chaxacterized natural plant compounds and extracts (natural disinfecting products) show antimicrobial activity when used in contact lens care products, oral care products, skin care products or for preserving an ophthalmic and other pharmaceutical and healthcare preparations. Not only are these natural compounds effective disinfecting and preservative agents, they have little or no potential to act as irritants when brought into contact with human ocular tissue. This is quite different from existing chemical antimicrobial agents used for these applications in that with the chemical antimicrobial agents there is a direct correlation between chemical agent concentration and irritation/discomfort of the treated tissue.

Of particular interest are the following natural antimicrobial agents, their derivatives and/or major constituent compounds: Allantoin, Berberine, Bilberry extract, Caffeic Acid Phenethyl Ether, Chlorogenic Acid, Cinnamaldehyde, Cranberry Extract, Elderberry Extract, Ferulic Acid, Gallic Acid and esters thereof (propyl gallate), Green Tea Extract, Grape Seed Extract, Hydroxytyrosol, Oleuropein, Olive Leaf Extract, Pine Bark Extract, Pomegranate Extract, Pycnogenol, Quercetin, Resveratrol, Tart Cherry Extract, Trans-Cinnamic Acid, Vanillic Acid, and/or combinations of the above. Our tests demonstrate that many of these natural products show significant abilities to act as disinfectants or preservatives. Of particular promise are Oleuropein, Gallic Acid, Cinmaldehyde, Green Tea Extract, Resveratrol, Trans-Cinnamic Acid, Pomegranate, Extract, Hydroxytyrosol and Cranberry Extract. Oleuropein, Green Tea Extract, Hydroxytyrosol, and Pomegranate Extract, either alone or in combination, and show special promise. The combinations of Vanillic Acid and Oleuropein and Propyl Gallate and Oleuropein are particularly effective combinations.

Several of the natural products can be combined to produce an extremely effective disinfecting/preservative solution with properties superior to a solution based on a single natural product Further, Allantoin, can be used to enhance the effectiveness of artificial disinfecting/preservative agents as well as to reduce the cytotoxicity of some synthetic chemical disinfecting/preservative agents The natural disinfecting and preservative agents show distinct advantages over the widely used synthetic chemical antimicrobial agents. In contrast to many of the synthetic agents that require EDTA to be effective, natural agents are effective without such additives. EDTA has been shown to have the potential to cause irritation to tissue. It would appear that the natural agents operate through different pathways than the synthetic chemical agents. The synthetic chemical agents tend to show cytotoxic effects and may damage the normal cells causing necrotic (uncontrolled) cell death as well as killing the microbes. On the other hand, natural agents often show antioxidant activity and are or can be protective of normal tissue cells while simultaneously showing antipathogen activity.

In the case of ophthalmic contact lens care products and disinfectants interactions of the disinfecting and preservative agents with the contact lens can cause unexpected magnifications of toxicity. Because with contact lenses there is absorption and adsorption of the disinfecting and preservative agents by the matrix of the contact lens polymer, which then can act as a reservoir to dose the eye with a continual stream of irritating chemical agents. The natural agents can have much less of a propensity to absorb and adsorb to the contact lens due to their chemical structure.

### DETAILED DESCRIPTION OF THE PREFERRED EMBODIMENTS

The following description is provided to enable any person skilled in the art to make and use the invention and sets forth the best modes contemplated by the inventors of carrying out their invention. Various modifications, however, will remain readily apparent to those skilled in the art, since the general principles of the present invention have been defined herein, specifically to provide an improved contact lens care solution and other pharmaceutical preparations based on naturally occurring plant compounds and extracts.

The following examples demonstrate the antimicrobial effectiveness of these naturally occurring plant compounds in typical ophthalmic and other topical formulations. In progressing through these examples one can understand the "building blocks" of the preferred embodiment of contact lens, ophthalmic solutions and pharmaceutical preparations incorporating these naturally occurring disinfecting materials.

In making the present invention a relatively long list of potential natural plant compounds was considered. This inclusive list is given below in Table 1

**Table 1**

| **Material/Compound** | **Class** | **Natural Source** |
|---|---|---|
| Allantoin | Purine derivative | Comfrey |
| Berberine | Alkaloid | Barberry, Golden Seal, Phylodendron |
| Bilberry extract | Anthocyanidin | Bilberry |
| Caffeic Acid | Phenolic acid | Many Fruits |
| Caffeic Acid Phenyl Ester (CAPE) | Caffeic Acid Derivative | Honey Bee Propolis |
| Chlorogenic Acid | Caffeic Acid Derivative | Fruits, Green Coffee Beans |
| Cinnamaldehyde | Aromatic Aldehyde | Cinnamon |
| Cranberry Extract | Proanthocyanidin | Cranberries |
| Elderberry Extract | Anthocyanidin | Elderberry |
| Ferulic Acid | Phenolic Acid | Pineapple |
| Gallic Acid | Phenolic Acid | Oak |
| Green Tea Extract | Catechin | Green Tea Leaves |
| Grape Seed Extract | Oligomeric Anthocyanidin | Grape Seed |
| Oleuropein | Phenolic Iridoid | Olive Leaf, Fruit & Oil |
| Olive Leaf Extract | Phenolic Iridoid | Olive Leaf |
| Pine Bark Extract | Oligomeric Anthocyanidin | Pine Bark |
| Pomegranate Extract | Extract/Polyphenol | Pomegranate |
| Propyl Gallate | Phenolic Acid Ester | Gallic Acid Derivative |
| Pycnogenol | Oligomeric Anthocyanidin | Pine Bark |
| Quercetin Dihydrate | Bioflavonoid | Oak |
| Resveratrol | Phenolic Stillbene | Red Grape Skins (red wine) |
| Tart Cherry Extract | Anthocyanidin | Sour Cherry |
| Trans-Cinnamic Acid | Aromatic Acid | Cinnamon |
| Vanillic Acid | Phenolic Acid | Vanilla |

In the case of materials derived from natural plant sources, particularly "extracts" a large number of active agents is probably present. The term "extract" is intended to indicate the presence of this mixture. The listed class is the group of compounds to which the major active agents of the extract are believed to belong. Nevertheless, it is likely that the extract contains a synergistic mixture of compounds in which activity may be contributed by compounds not of the class mentioned in the table.

Various compounds/extracts were tested in the presence of either a phosphate or a borate-buffered solution. The test organisms included (S. aureus. = *Staphylococcus aureus;* Ps. aeruginosa = *Pseudomonas aeruginosa,* E. coli = *Escherichia coli; C.* albicans. = *Candida albicans;* and A. *niger = Aspergillus niger*) and inspected at various time intervals to determine the "log kill" of the various formulations, Table 2 shows the results for nine of the test compounds.

**Table 2.¹**

| In Borate Buffered Saline | | | | | | | | | |
|---|---|---|---|---|---|---|---|---|---|
| | **Formulae (ppm)** | | | | | | | | |
| **Ingredient** | **A** | **B** | **C** | **D** | **E** | **F** | **G** | **H** | **I** |
| Berberine sulfate | 200 | 100 | 50 | 20 | 0 | 0 | 0 | 0 | 0 |
| Allantoin | 0 | 0 | 0 | 0 | 1000 | 500 | 250 | 100 | 0 |
| Olive Leaf Extract² | 0 | 0 | 0 | 0 | 0 | 0 | 0 | 0 | 1000 |

| **Log Kill (1 Day)** | | | | | | | | | |
|---|---|---|---|---|---|---|---|---|---|
| S. aureus | 0.3 | 0.3 | 0.2 | 0.1 | 0.0 | 0.2 | 0.2 | 0.0 | 0.3 |
| Ps. aeruginosa | 1.5 | 1.4 | 1.1 | 0.6 | 0.4 | 0.3 | 0.7 | 0.7 | 4.1 |
| E. coli | 0.0 | 0.1 | 0.0 | 0.0 | 0.1 | 0.1 | 0.1 | 0.1 | 0.2 |
| C. albicans | 0.2 | 0.1 | 0.1 | 0.2 | 0.1 | 0.0 | 0.1 | 0.2 | 0.7 |
| A niger | 1.6 | 1.5 | 1.6 | 1.8 | 1.5 | 1.6 | 1.4 | 0.7 | 1.4 |

| **Log Kill (7 Days)** | | | | | | | | | |
|---|---|---|---|---|---|---|---|---|---|
| S. aureus | ≥5.2 | ≥5.2 | ≥5.2 | ≥5.2 | 0.2 | 0.2 | 0.6 | 0.9 | ≥5.2 |
| Ps. aeruginosa | 0.7 | 0.5 | 0.4 | 0.3 | 0.2 | 0.2 | 0.3 | 0.1 | 4.1 |
| E. coli | 0.2 | 0.1 | 0.0 | 0.2 | 0.3 | 0.4 | 0.4 | 0.4 | 1.1 |
| C. albicans | ≥5.2 | ≥5.2 | ≥5.2 | 4.7 | ≥5.2 | 4.3 | ≥5.2 | 5.2 | 3.2 |
| A.niger | 3.2 | 3.8 | 3.3 | 3.3 | 3.4 | 3.4 | 4.0 | 4.2 | 3.1 |

| **Log Kill (14 Days)** | | | | | | | | | |
|---|---|---|---|---|---|---|---|---|---|
| S. aureus | ≥5.2 | ≥5.2 | ≥5.2 | ≥5.2 | 1.3 | 1.4 | 1.7 | 2.2 | ≥5.2 |
| Ps. aeruginosa | 0.3 | 0.4 | 0.3 | 0.5 | 0.4 | 0.3 | 0.2 | 0.2 | ≥5.1 |
| E. coli | 0.2 | 0.4 | 0.3 | 0.0 | 0.2 | 0.2 | 0.3 | 0.3 | 1.1 |
| C. albicans | ≥5.2 | ≥5.2 | ≥5.2 | 4.7 | ≥5.2 | 4.3 | ≥5.2 | 5.2 | 3.2 |
| A. niger | 3.2 | 3.8 | 3.3 | 3.3 | 3.4 | 3.4 | 4.0 | 4.2 | 3.1 |

| **Log Kill (28 Days)** | | | | | | | | | |
|---|---|---|---|---|---|---|---|---|---|
| S. aureus | N.T. | N.T. | N.T. | N.T. | N.T. | N.T. | N.T | N.T. | ≥5.2 |
| Ps. aeruginosa | N.T. | N.T. | N.T. | N.T. | N.T. | N.T. | N.T. | N.T. | ≥5.1 |
| E.coli | N.T. | N.T. | N.T. | N.T. | N.T. | N.T. | N.T. | N.T. | 1.1 |
| C. albicans | N.T. | N.T. | N.T. | N.T. | N.T. | N.T. | N.T. | N.T. | 3.2 |
| A. niger | N.T. | N.T. | N.T. | N.T. | N.T. | N.T. | N.T. | N.T. | 3.1 |

| | | | | | | | | | |
|---|---|---|---|---|---|---|---|---|---|
| ¹ N.T.="not tested" ² Olive leaf extract not totally soluble; dissolved in hot solution and filtered. | | | | | | | | | |

It can be seen that Berberine is remarkably effective against *Staphylococcus, Candida* and *Aspergillus* even at quite low concentrations. Allantoin is quite effective against *Candida* and *Aspergillus,* and in other tests was shown (see below) to protect against eye irritation caused by chemical disinfectant agents. Olive Leaf Extract shows a range of effectiveness and is most effective against *Staphylococcus* and *Pseudomonas* although it also shows considerable efficacy against *Candida* and *Aspergillus.* The fungi are often resistant to chemical disinfecting agents but show significant susceptibility to natural agents of plant origin. The continued effectiveness of olive leaf extract over a 28-day period shows that this material is an effective preservative.

Tests of an additional set of candidate natural product preservatives/disinfectants in borate buffered saline are shown in Table 3. The same test protocols were followed as for the previous Table.

**Table 3.**

| In Borate Buffered Saline | | | | | | | | |
|---|---|---|---|---|---|---|---|---|
| | **Formulae (ppm)** | | | | | | | |
| **Ingredient** | **J** | **K** | **L** | **M** | **N** | **O** | **P** | **Q** |
| Bilberry Extract A 95% | 1000 | 0 | 0 | 0 | 0 | 0 | 0 | 0 |
| Bilberry Extract B 25% | 0 | 1000 | 0 | 0 | 0 | 0 | 0 | 0 |
| Tart Cherry Extract | 0 | 0 | 1000 | 0 | 0 | 0 | 0 | 0 |
| Elderberry Extract 15.2% | 0 | 0 | 0 | 1000 | 0 | 0 | 0 | 0 |
| Pomegranate Extract | 0 | 0 | 0 | 0 | 1000 | 0 | 0 | 0 |
| Querectin Dihydrate | 0 | 0 | 0 | 0 | 0 | 1000 | 0 | 0 |
| CAPE | 0 | 0 | 0 | 0 | 0 | 0 | 1000 | 0 |
| Resveratrol (25%) | 0 | 0 | 0 | 0 | 0 | 0 | 0 | 1000 |

| **Log Kill (1 Day)** | | | | | | | | |
|---|---|---|---|---|---|---|---|---|
| S. aureus | 3.5 | 0.2 | 0.1 | 0.3 | 1.2 | 0.3 | 0.7 | 3.9 |
| Ps. aeruginosa | 5.0 | 1.2 | 1.1 | 3.1 | 4.3 | 5.0 | 3.2 | ≥5.0 |
| E. coli | 1.1 | 0.2 | 0.1 | 1.3 | 2.2 | 1.0 | 0.3 | 2.3 |
| C. albicans | 0.4 | 0.5 | 0.6 | 0.7 | 1.0 | 0.2 | 0.4 | 2.0 |
| A. niger | 0.1 | 1.1 | 0.4 | 0.2 | 0.1 | 0.1 | 0.0 | 0.0 |

| **Log Kill (7 Days)** | | | | | | | | |
|---|---|---|---|---|---|---|---|---|
| S. aureus | ≥5.0 | 1.8 | 1.6 | 3.8 | ≥5.0 | ≥5.0 | ≥5.0 | ≥5.0 |
| Ps. aeruginosa | ≥5.0 | 4.3 | ≥5.0 | ≥5.0 | ≥5.0 | ≥5.0 | ≥5.0 | ≥5.0 |

| | **Formulae (ppm)** | | | | | | | |
|---|---|---|---|---|---|---|---|---|
| **Ingredient** | **J** | **K** | **L** | **M** | **N** | **O** | **P** | **Q** |
| E. coli | ≥5.2 | 0.8 | 0.6 | 0.5 | ≥5.2 | ≥5.2 | 5.2 | ≥5.2 |
| C. albicans | 2.1 | 1.7 | 2.3 | 0.5 | ≥5.1 | 3.7 | ≥5.1 | ≥5.1 |
| A. niger | 2.8 | 2.3 | 3.0 | 1.8 | 2.5 | 2.5 | 2.1 | 1.2 |

| **Log Kill (14 Days)** | | | | | | | | |
|---|---|---|---|---|---|---|---|---|
| S. aureus | ≥5.0 | 4,3 | 3.5 | ≥5.0 | >5.0 | ≥5.0 | ≥5.0 | ≥5.0 |
| Ps. aeruginosa | ≥5.0 | ≥5.0 | ≥5.0 | ≥5.0 | ≥5.0 | ≥5.0 | ≥5.0 | ≥5.0 |
| E.coli | ≥5.2 | 0.7 | 0.5 | 1.6 | ≥5.2 | ≥5.2 | ≥5.2 | ≥5.2 |
| C. albicans | 3.0 | 2.7 | 3.1 | 2.7 | ≥5.1 | ≥5.1 | ≥5.1 | ≥5.1 |
| A. niger | 3.3 | 3.1 | 3.3 | 3.1 | 2.5 | 3.1 | 2.2 | 1.1 |

| **Log Kill (28 Days)** | | | | | | | | |
|---|---|---|---|---|---|---|---|---|
| S. aureus | ≥5.0 | ≥5.0 | ≥5.0 | ≥5.0 | ≥5.0 | ≥5.0 | ≥5.0 | ≥5.0 |
| Ps. aeruginosa | ≥5.0 | ≥5.0 | 3.4 | ≥5.0 | ≥5.0 | ≥5.0 | ≥5.0 | ≥5.0 |
| E. coli | ≥5.2 | 0.3 | 0.2 | 1.7 | ≥5.2 | ≥5.2 | ≥5.2 | ≥5.2 |
| C. albicans | ≥5.1 | 5.1 | ≥5.1 | ≥5.1 | ≥5.1 | ≥5.1 | ≥5.1 | ≥5.1 |
| A. nigger | 3.2 | 2.8 | 2.9 | 2.9 | 2.4 | 2.7 | 2.0 | 3.2 |

Bilberry Extract A shows broad effectiveness against all the test organisms with it being the most effective against the bacteria. Bilberry Extract B (a more dilute extract) shows lesser effectiveness particularly against *S. aureus* and *E. coli.* Over the 28 days of the test Bilberry Extract B gradually "catches up" on some organisms but not on *E. coli.* Tart Cherry Extract also shows broad effectiveness but like Bilberry Extract B appears less effective against *E. coli.* This same pattern is also seen with the Elderberry Extract. On the other hand, Pomegranate Extract is extremely effective against all the test organisms. Quercetin dihydrate also shows extremely broad effectiveness. Since Quercetin is a component of many fruits, it is tempting to hypothesize that it may be a major active component of several fruit extracts. CAPE, a component of honey also shows good activity, particularly at time greater than one day. Finally, resveratrol, another polyphenolic compound found in grapes, also shows a wide range of effectiveness. Any of these agents are excellent long-term preservatives.:

Table 4 shows results of additional natural ingredients tested in borate buffered saline including green tea extracts and grape seed extracts from multiple sources to investigate source-to-source variability.

**Table 4.**

| In Borate Buffered Saline | | | | | | | |
|---|---|---|---|---|---|---|---|
| | **Formulae (ppm)** | | | | | | |
| **Ingredient** | **R** | **S** | **T** | **U** | **V** | **W** | **X** |
| Green Tea Extract A 50%EGCG | 1000 | 0 | 0 | 0 | 0 | 0 | 0 |
| Green Tea Extract B 93%EGCG | 0 | 1000 | 0 | 0 | 0 | 0 | 0 |
| Grape Seed Extract A 95% | 0 | 0 | 1000 | 0 | 0 | 0 | 0 |
| Grape Seed Extract B | 0 | 0 | 0 | 1000 | 0 | 0 | 0 |
| Grape Seed Extract C | 0 | 0 | 0 | 0 | 1000 | 0 | 0 |
| Ferric Acid | 0 | 0 | 0 | 0 | 0 | 1000 | 0 |
| Chlorogenic Acid | 0 | 0 | 0 | 0 | 0 | 0 | 1000 |
| | | | | | | | |

| **Log kill (1 Day)** | | | | | | | |
|---|---|---|---|---|---|---|---|
| S. aureus | 1.3 | 2.8 | 0.7 | 0.9 | 0.9 | 3.4 | 1.7 |
| Ps. aeruginosa | ≥5.1 | ≥5.1 | 4.3 | 4.3 | 2.2 | ≥4.9 | 3.9 |
| E. coli | 1.3 | 4.3 | 1.1 | 0.7 | 0.8 | 3.5 | 0.1 |
| C. albicans | 0.7 | 2.4 | 0.3 | 0.8 | 0.4 | 2.0 | 0.2 |
| A. niger | 1.1 | 1.1 | 0.9 | 1.0 | 0.9 | 1.1 | 1.7 |

| **Log Kill (7 Days)** | | | | | | | |
|---|---|---|---|---|---|---|---|
| S. aureus | ≥5.0 | ≥5.0 | 4.3 | ≥5.0 | 4.2 | ≥5.1 | ≥5.1 |
| Ps. aeruginosa | ≥5.1 | ≥5.1 | ≥5.1 | ≥5.1 | 2.5 | ≥4.9 | ≥4.9 |
| E. coli | ≥5.4 | ≥5.4 | 2.7 | ≥5.4 | 1.4 | ≥5.1 | ≥4.4 |
| C. albicans | 2.1 | 5.0 | 0.9 | 3,7 | 0.8 | ≥5.1 | 2.5 |
| A. niger | 2.7 | 2.7 | 2.0 | 2.6 | 2.0 | 1.6 | 2.2 |

| **Log Kill (14 Days)** | | | | | | | |
|---|---|---|---|---|---|---|---|
| S. aureus | ≥5.0 | ≥5.0 | ≥5.0 | ≥5.0 | ≥5.0 | ≥5.1 | ≥5.1 |
| Ps. aeruginosa | ≥5.1 | ≥5.1 | ≥5.1 | ≥5.1 | ≥5.1 | ≥4.9 | ≥4.9 |
| E. coli | ≥5.4 | ≥5.4 | 1.0 | ≥5.4 | 1.5 | ≥5.1 | ≥5.1 |
| C. albicans | 3.4 | ≥5.0 | 2.0 | ≥5.0 | 3.9 | ≥5.1 | ≥5.1 |
| A. niger | 3.1 | 2.9 | 2.9 | 2.3 | 2.5 | 1.6 | 1.3 |
| **Log Kill (28 Days)** | | | | | | | |

| | **Formulae (ppm)** | | | | | | |
|---|---|---|---|---|---|---|---|
| **Ingredient** | **R** | **S** | **T** | **U** | **V** | **W** | **X** |
| S. aureus | ≥5.0 | ≥5.0 | ≥5.0 | ≥5.0 | ≥5.0 | ≥5.1 | ≥5.1 |
| Ps. aeruginosa | ≥5.1 | ≥5.1 | ≥5.0 | ≥5.1 | ≥5.1 | ≥4.9 | ≥4.9 |
| E. coli | ≥5.4 | ≥5.4 | 1.6 | ≥5.4 | 1.5 | ≥5.1 | ≥5.1 |
| C. albicans | ≥5.0 | ≥5.0 | 4,3 | ≥5.0 | 3.9 | ≥5.1 | ≥5.1 |
| A. niger | 2.7 | 2.7 | 2.7 | 2.0 | 2.5 | 1.0 | 1.3 |

Green Tea Extract B is remarkably effective even at the day one time point. As might be expected, it is somewhat more effective than the less concentrated Green Tea Extract A. The three different Grape Seed Extracts are relatively similar. However, Grape Seed Extract B is significantly more effective against *E. coli* and *C. albicans* than the other two. Similarly Extract C is less effective against *Pseudomonas* than are the other two extracts. This suggests that the extracts all contain a spectrum of active ingredient—some ingredients being active against one test organism, some against another—and that the exact level of each active ingredient can vary from extract to extract. Chlorogenic acid and Ferulic acid are also quite effective with Ferulic acid showing better one day activity and better activity against *C. albicans.*

Additional experiments were performed to formulate some of the natural ingredients with a wetting agent (Pluronic F-68) as might be found in an ophthalmic preparation. The results for Quercetin and CAPE in borate buffered saline are shown in the following Table 5.

**Table 5.**

| In Borate Buffered Saline | | | | | | | | | |
|---|---|---|---|---|---|---|---|---|---|
| | **Formulae (ppm)** | | | | | | | | |
| **Ingredient** | **AA** | **BB** | **cc** | **DD** | **EE** | **FF** | **GG** | **HH** | **II** |
| Quercetin Dihydrate | 1000 | 500 | 200 | 50 | 0 | 0 | | | 0 |
| CAPE | 0 | 0 | 0 | 0 | 700 | 400 | 200 | 50 | 500 |

| | **Formulae (ppm)** | | | | | | | | |
|---|---|---|---|---|---|---|---|---|---|
| **Ingredient** | **AA** | **BB** | **CC** | **DD** | **EE** | **OFF** | **GG** | **HH** | **II** |
| Pluronic F-68 | 0 | 0 | 0 | 0 | 1000 | 0 | | | 1000 |
| | | | | | | | | | |

| **Log Kill (1 Day)** | | | | | | | | | |
|---|---|---|---|---|---|---|---|---|---|
| S. aureus | 1.3 | 1.9 | 1.3 | 0.3 | 4.3 | 3.4 | 2.7 | 1.3 | 1.2 |
| Ps. aeruginosa | ≥4.9 | ≥4.9 | 4,4 | 2.5 | ≥5.0 | 5.0 | 3.2 | 2.7 | 4.7 |
| E.coli | 0.4 | 0.4 | 0.3 | 0.0 | 1.2 | 0.1 | 0.1 | 0.1 | 0.3 |
| C. albicans | 0.4 | 0.5 | 0.3 | 0.0 | 0.8 | 0.6 | 0.3 | 0.2 | 0.5 |
| A. niger | 1.5 | 0.9 | 1.7 | 2.4 | 1.5 | 1.5 | 1.3 | 1.6 | 1.4 |

| **Log Kill (7 Days)** | | | | | | | | | |
|---|---|---|---|---|---|---|---|---|---|
| S.aureus | ≥5.1 | ≥5.1 | ≥5.1 | 5.1 | ≥5.0 | ≥5.0 | ≥5.0 | 5.0 | ≥5.0 |
| Ps. aeruginosa | ≥4.9 | ≥4.9 | ≥4.9 | ≥4.9 | ≥5.0 | ≥5.0 | ≥5.0 | ≥5.0 | ≥5.0 |
| E.coli | ≥5.1 | ≥5.1 | 3.7 | 0.9 | ≥5.1 | 3.7 | 0.5 | 0.6 | 1.1 |
| C. albicans | 3.2 | 3.4 | 2.5 | 0.9 | 4.4 | 2.7 | 1.8 | 1.2 | 1.7 |
| A. niger | 1.7 | 1.7 | 1.6 | 1.9 | 2.4 | 2.7 | 2.5 | 2.6 | 2.5 |

| **LogKill (14Days)** | | | | | | | | | |
|---|---|---|---|---|---|---|---|---|---|
| S.aureus | ≥5.1 | ≥5.1 | ≥5.1 | ≥5.1 | ≥5.0 | ≥5.0 | ≥5.0 | ≥5.0 | ≥5.0 |
| Ps. aeruginosa | ≥4.9 | ≥4.9 | ≥4.9 | ≥4.9 | ≥5.0 | ≥5.0 | ≥5.0 | ≥5.0 | ≥5.0 |
| E.coli | ≥5.1 | ≥5.1 | ≥5.1 | ≥5.1 | ≥5.1 | ≥5.1 | 3.1 | 1.2 | 1.8 |
| C. albicans | ≥5.1 | ≥5.1 | ≥5.1 | 3.0 | ≥5.0 | 4.5 | 3.3 | 2.4 | 2.8 |
| A. nigger | 1.7 | 1.8 | 2.9 | 1.9 | 2.9 | 4.0 | 2.7 | 2.6 | 2.7 |

| **Log Kill (28 Days)** | | | | | | | | | |
|---|---|---|---|---|---|---|---|---|---|
| S. aureus | ≥5.1 | ≥5.1 | ≥5.1 | ≥5.1 | ≥5.0 | ≥5.0 | ≥5.0 | ≥5.0 | ≥5.0 |
| Ps. aeruginosa | ≥4.9 | ≥4.9 | ≥4.9 | ≥4.9 | ≥5.0 | ≥5.0 | ≥5.0 | ≥5.0 | ≥5.0 |
| E.coli | ≥5.1 | ≥5.1 | ≥5.1 | ≥5.1 | ≥5.1 | ≥5.1 | ≥5.0 | 3.5 | 3.8 |
| C. albicans | ≥5.1 | ≥5.1 | ≥5.1 | ≥5.1 | ≥5.0 | ≥5.0 | ≥5.0 | ≥5.0 | ≥5.0 |
| A. niger | 1.4 | 1.2 | 1.2 | 1.3 | 4.5 | 4.0 | 3.0 | 1.6 | 2.2 |

Both Quercetin and CAPE in the presence of the Pluronic F-68 show very good to excellent control of the test microbes at times greater than one day.

Similarly, additional experiments were undertaken to test the two different Bilberry and two different green tea extracts in borate buffered saline at various concentrations with the above-described borate buffers. The results are shown in the following Table 6.

**Table 6.**

| In Borate Buffered Saline | | | | | | | | | | |
|---|---|---|---|---|---|---|---|---|---|---|
| | **Formulae (ppm)** | | | | | | | | | |
| **Ingredient** | **JJ** | **KK** | **LL** | **MM** | **NlW** | **00** | **pp** | **QQ** | **RR** | **SS** |
| Bilberry Extract A (95%) | 1000 | 500 | 200 | 50 | 0 | 0 | | | | 0 |
| Bilberry Exact B (25%) | 0 | 0 | 0 | 0 | 200 | 0 | 0 | 0 | | 0 |
| Green Tea Extract B | 0 | 0 | 0 | 0 | | 1000 | 500 | 200 | 50 | 0 |
| Green Tea Extract C | | | | | | | | | | 1000 |
| | | | | | | | | | | |

| **Log Kill (1 Day)** | | | | | | | | | | |
|---|---|---|---|---|---|---|---|---|---|---|
| S.aureus | 1.0 | 0.2 | 0.5 | 0 | 0.2 | 5.0 | 3.9 | 2.9 | 1.6 | 2.2 |
| Ps. aeruginosa | ≥4.9 | ≥4.9 | 3.8 | 2.4 | 3.3 | ≥4.9 | ≥4.9 | ≥4.9 | ≥4.9 | ≥4.9 |
| E. coli | 0.6 | 0.2 | 0 | 0.1 | 0.2 | 3.9 | 2.4 | 1.4 | 0.2 | 4.2 |
| C. albicans | 0.5 | 0.9 | 0.6 | 0.1 | 0.6 | 1.1 | 1.1 | 1.5 | 0.5 | 1.4 |
| A.niger | 1.6 | 1.6 | 1.9 | 2.0 | 1.7 | 1.7 | 1.6 | 1.9 | 1.9 | 1.8 |

| **Log Kill (7 Days)** | | | | | | | | | | |
|---|---|---|---|---|---|---|---|---|---|---|
| S. aureus | ≥5.1 | ≥5.1 | ≥5.1 | 5.1 | 3.4 | ≥5.1 | ≥5.1 | ≥5.1 | ≥5.1 | ≥5.1 |
| Ps. aeruginosa | >4.9 | ≥4.9 | 4.9 | k4.9 | ≥4.9 | ≥4.9 | ≥4.9 | ≥4.9 | ≥5.1 | ≥4.9 |
| E. coli | ≥5.1 | ≥5.1 | 2.1 | 0.2 | 1.1 | ≥5.1 | ≥5.1 | ≥5.1 | ≥5.1 | ≥5.1 |
| C. albicans | 1.8 | 2.2 | 1.7 | 1.9 | 2.0 | 4.8 | ≥5.1 | ≥5.1 | 3.3 | ≥5.1 |
| A. niger | 2.5 | 1.8 | 1.7 | 2.0 | 2.4 | 1.7 | 1.7 | 2.3 | 2.0 | 1.4 |

| **Log Kill (14 Days)** | | | | | | | | | | |
|---|---|---|---|---|---|---|---|---|---|---|
| S. aureus | ≥5.1 | ≥5.1 | ≥5.1 | ≥5.1 | ≥5.1 | ≥5.1 | ≥5.1 | ≥5.1 | ≥5.1 | ≥5.1 |
| Ps. aeruginosa | ≥4.9 | ≥4.9 | ≥4.9 | ≥4.9 | ≥4.9 | ≥4.9 | ≥4.9 | ≥4.9 | ≥4.9 | ≥4.9 |
| E. coli | ≥5.1 | ≥5.1 | 5.1 | 0.4 | 0.6 | ≥5.1 | ≥5.1 | ≥5.1 | ≥5.1 | ≥5.1 |
| C. albicans | 2.8 | 3.1 | 2.5 | 2.7 | 3.5 | >5.1 | ≥5.1 | ≥5.1 | 4.8 | ≥5.1 |
| A. niger | 2.9 | 2.8 | 1.8 | 1.9 | 2.8 | 1.5 | 1.7 | 2.7 | 1.8 | 1.5 |

| **Log Kill (28 Days)** | | | | | | | | | | |
|---|---|---|---|---|---|---|---|---|---|---|
| S. aureus | ≥5.1 | ≥5.1 | ≥5.1 | ≥5.1 | ≥5.1 | ≥5.1 | ≥5.1 | ≥5.1 | ≥5.1 | ≥5.1 |
| Ps. aeruginosa | ≥4.9 | ≥4.9 | ≥4.9 | ≥4.9 | ≥4.9 | ≥4.9 | ≥4.9 | ≥4.9 | ≥4.9 | ≥4.9 |
| E. coli | ≥5.1 | ≥5.1 | ≥5.1 | 0 | 0.9 | ≥5.1 | ≥5.1 | ≥5.1 | ≥5.1 | ≥5.1 |
| C.albicans | ≥5.1 | ≥5.1 | ≥5.1 | ≥5.1 | ≥5.1 | ≥5.1 | ≥5.1 | ≥5.1 | ≥5.1 | ≥5.1 |
| A. niger | 2.4 | 2.2 | 1.5 | 1.6 | 2.8 | 1.2 | 1.3 | 1.3 | 1.4 | 0.5 |

Again, both Bilberry and green tea show excellent disinfectant and preservative properties at all test times with green tea being strikingly effective at one day.

Similarly, additional experiments were undertaken to retest pomegranate at various concentrations as well as oleuropein and grape seed extract with the above-described borate buffer. In addition, two different pine bark extracts (U.S. pine bark extract and pycnogenol) were tested using the same buffer. The results are shown in the following Table 7.

**Table 7.**

| In Borate Buffered Saline | | | | | | | | |
|---|---|---|---|---|---|---|---|---|
| | **Formulae (ppm)** | | | | | | | |
| **Ingredient** | **TT** | **UU** | **VV** | **WW** | **XX** | **YY** | **ZZ** | **A3** |
| Pine Bark Extract (USA) | 1000 | 0 | 0 | 0 | 0 | 0 | 0 | 0 |
| Pycnogenol (Europe) | 0 | 1000 | 0 | 0 | 0 | 0 | 0 | 0 |
| Pomegranate Extract | 0 | 0 | 1000 | 500 | 200 | 50 | 0 | 0 |
| Oleuropein | 0 | 0 | 0 | 0 | 0 | 0 | 1000 | 0 |
| Grape Seed Polyphenolics | 0 | 0 | 0 | 0 | 0 | 0 | 0 | 1000 |
| | | | | | | | | |

| **Log KH1 (1 Day)** | | | | | | | | |
|---|---|---|---|---|---|---|---|---|
| S. aureus | 0.5 | 3.5 | 1.9 | 1.4 | 0.7 | 0 | 4.2 | 2.0 |
| Ps. aeruginosa | k4.9 | 4.9 | ≥4.7 | ≥4.7 | ≥4.7 | 2.7 | ≥5.0 | ≥4.7 |
| E. coli | 0.9 | 0.4 | 0.5 | 0.4 | 0.2 | 0 | 1.9 | 0.9 |
| C. albicans | 0.6 | 1.6 | 1.1 | 0.8 | 0.5 | 0.2 | 1.1 | 1.1 |
| A. niger | 1.9 | 0.9 | 2.9 | 3.0 | 2.9 | 3.0 | 2.3 | 1.9 |

| **Log Kill (7Days)** | | | | | | | | |
|---|---|---|---|---|---|---|---|---|
| S. aureus | ≥5.1 | ≥5.1 | ≥4.9 | ≥4.9 | ≥4.9 | 2.9 | ≥5.0 | ≥4.9 |
| Ps. aeruginosa | ≥4.9 | ≥4.9 | ≥4.7 | ≥4.7 | ≥4.7 | ≥4.7 | ≥5.0 | ≥4.7 |
| E. coli | ≥5.1 | ≥5.1 | ≥5.0 | ≥5.0 | 3.8 | 0.9 | ≥5.1 | ≥5.0 |
| C. albicans | 1.9 | 3.1 | ≥5.0 | 4.4 | 3.2 | 2.1 | 3.1 | 5.0 |
| A. niger | 1.7 | 1.6 | 2.1 | 2.7 | 2.7 | 2.7 | 2.7 | 1.8 |

| **Log Kill (14 Days)** | | | | | | | | |
|---|---|---|---|---|---|---|---|---|
| S. aureus | ≥5.1 | ≥5.1 | ≥4.9 | ≥4.9 | ≥4.9 | ≥4.9 | ≥5.0 | ≥4.9 |
| Ps. aeruginosa | ≥4.9 | ≥4.9 | ≥4.7 | ≥4.7 | ≥4.7 | ≥4.7 | ≥5.0 | ≥4.7 |
| E.coli | ≥5.1 | ≥5.1 | ≥5.0 | ≥5.0 | ≥5.0 | 2.2 | ≥5.1 | ≥5.0 |
| C. albicans | 2.9 | 3.9 | ≥5.0 | ≥5.0 | ≥5.0 | ≥5.0 | ≥5.0 | ≥5.0 |
| A. niger | 1.8 | 1.7 | 1.8 | 1.9 | 2.8 | 1.5 | 1.7 | 2.7 |
| **Log Kill (28 Days)** | | | | | | | | |

| | **Formulae (ppm)** | | | | | | | |
|---|---|---|---|---|---|---|---|---|
| **Ingredient** | **TT** | **UU** | **VV** | **WW** | **XX** | **YY** | **ZZ** | **A3** |
| S.aureus | ≥5.1 | ≥5.1 | ≥4.9 | ≥4.9 | ≥4.9 | ≥4.9 | ≥5.0 | ≥4.9 |
| Ps. aeruginosa | ≥4.9 | ≥4.9 | ≥4.7 | ≥4.7 | ≥4.7 | ≥4.7 | ≥5.0 | ≥4.7 |
| E. coli | ≥5.1 | ≥5.1 | ≥5.0 | ≥5.0 | ≥5.0 | ≥5.0 | ≥5.1 | 5.0 |
| C. albicans | ≥5.1 | ≥5.1 | ≥5.0 | ≥5.0 | ≥5.0 | ≥5.0 | ≥5.0 | ≥5.0 |
| A. niger | 0.8 | 1.6 | 1.4 | 1.4 | 1.4 | 1.4 | 1.6 | 1.4 |

Table 8 shows additional experiments with Resveratrol (from *Polygonum cuspidatum*), oleuropein and grape seed extract in the above-described borate buffer.

**Table 8.**

| In Borate Buffered Saline | | | | | | | | | |
|---|---|---|---|---|---|---|---|---|---|
| | **Formulae (ppm)** | | | | | | | | |
| | **B3** | **C3** | **D3** | **E3** | **F3** | **G3** | **H3** | **I3** | **J3** |
| **Ingredient** | | | | | | | | | |
| Grape seed Extract | 1000 | 500 | 200 | 50 | 0 | 0 | 0 | | |
| Resveratrol (*Polygonum cuspidatum*) | 0 | 0 | 0 | 0 | 500 | 0 | 0 | | |
| Oleuropein | 0 | 0 | 0 | 0 | | 1000 | 500 | 200 | 50 |
| | | | | | | | | | |
| **pH of solution** | 7.2-7.6 | 7.2-7.7 | 7.2-7.8 | 7.2-7.9 | 7.2-7.6 | 7.2-7.6 | 7.2-7.6 | 7.2-7.6 | 7.2-7.6 |
| | | | | | | | | | |

| **Log Kill (1 Day)** | | | | | | | | | |
|---|---|---|---|---|---|---|---|---|---|
| S. aureus | 4.0 | 3.7 | 1.1 | 0.7 | 2.9 | 1.6 | 1.0 | 0.3 | 0.1 |
| Ps. aeruginosa | ≥4.9 | ≥4.9 | ≥4.7 | 3.1 | 3.9 | ≥4.8 | ≥4.8 | ≥4.8 | 3.4 |
| E. coli | 2.0 | 0.5 | 0.1 | 0.1 | 0.2 | 0.6 | 0.4 | 0.2 | 0.1 |
| C. albicans | 2.5 | 1.4 | 1.0 | 0.3 | 0.5 | 0.8 | 0.9 | 0.8 | 0.3 |
| A. niger | 1.7 | 1.8 | 1.8 | 1.6 | 1.8 | 1.4 | 1.8 | 1.8 | 1.8 |

| **Log Kill (7 Days)** | | | | | | | | | |
|---|---|---|---|---|---|---|---|---|---|
| S. aureus | ≥5.0 | ≥5.0 | ≥5.0 | ≥5.0 | ≥5.0 | ≥5.0 | ≥5.0 | ≥5.0 | ≥5.0 |
| Ps. aeruginosa | ≥4.9 | ≥4.9 | ≥4.9 | ≥4.9 | ≥4.9 | ≥4.8 | ≥4.8 | ≥4.8 | ≥4.8 |
| E. coli | ≥5.2 | ≥5.2 | 3.5 | 0.4 | ≥5.2 | ≥5.3 | ≥5.3 | 4.6 | 1.7 |

| | **Formulae (ppm)** | | | | | | | | |
|---|---|---|---|---|---|---|---|---|---|
| | **B3** | **C3** | **D3** | **E3** | **F3** | **G3** | **H3** | **13** | **J3** |
| C. albicans | ≥5.0 | ≥5.0 | 4.5 | 3.2 | 5.0 | 3.4 | 3.5 | 2.8 | 3.0 |
| A. niger | 1.9 | 1.8 | 1.7 | 1.7 | 1.7 | 2.0 | 2.5 | 2.5 | 2.5 |

| **Log Kill (14 Days)** | | | | | | | | | |
|---|---|---|---|---|---|---|---|---|---|
| S. aureus | ≥5.0 | ≥5.0 | ≥5.0 | ≥5.0 | ≥5.0 | ≥5.0 | ≥5.0 | ≥5.0 | ≥5.0 |
| Ps. aeruginosa | ≥4.9 | ≥4.9 | ≥4.9 | ≥4.9 | ≥4.9 | ≥4.8 | ≥4.8 | ≥4.8 | ≥4.8 |
| E. coli | ≥5.2 | ≥5.2 | ≥5.2 | 1.2 | ≥5.2 | ≥5.3 | ≥5.3 | ≥5.3 | 1.7 |
| C. albicans | ≥5.0 | ≥5.0 | 5.0 | ≥5.0 | ≥5.0 | ≥5.1 | 5.1 | 5.1 | 5.1 |
| A. niger | 1.5 | 1.5 | 1.6 | 1.5 | 1.7 | 1.8 | 1.8 | 1.7 | 1.5 |

| **Log Kill (28 Days)** | | | | | | | | | |
|---|---|---|---|---|---|---|---|---|---|
| S. aureus | ≥5.0 | ≥5.0 | ≥5.0 | ≥5.0 | ≥5.0 | NT | NT | NT | NT |
| Ps. aeruginosa | ≥4.9 | ≥4.9 | ≥4.9 | ≥4.9 | ≥4.9 | NT | NT | NT | NT |
| E. coli | ≥5.2 | ≥5.2 | ≥5.2 | 1.8 | ≥5.2 | NT | NT | NT | NT |
| C. albicans | ≥5.0 | ≥5.0 | ≥5.0 | ≥5.0 | ≥5.0 | NT | NT | NT | NT |
| A. nigger | 1.3 | 1.3 | 0.9 | 1.2 | 1.4 | NT | NT | NT | NT |

| | | | | | | | | | |
|---|---|---|---|---|---|---|---|---|---|
| NT = Not Tested. | | | | | | | | | |

The various compounds all showed excellent activity at times greater than one day. Additional experiments were undertaken to test additional Resveratrol at a variety of concentrations, as well as Bilberry extract and Cranberry extract (at a low concentration) in borate buffered saline. Partial results on performance of hydroxytyrosol (3,4-dihydroxy-phenylethanol), a potent anti-oxidant found in olive oil, are also included. These results are shown in the following Table 9.

**Table 9.**

| In Borate Buffered Saline | | | | | | | |
|---|---|---|---|---|---|---|---|
| | **Formulae (ppm)** | | | | | | |
| **Ingredient** | **K3** | **L3** | **M3** | **N3** | **03** | **P3** | **Q3** |
| Resveratrol | 1000 | 500 | 200 | 50 | 0 | 0 | |
| Bilberry Extract (European) | 0 | 0 | 0 | 0 | 1000 | 0 | |
| Cranberry Extract | 0 | 0 | 0 | 0 | 0 | 120 | |
| Hydroxytyrosol | 0 | 0 | 0 | 0 | 0 | 0 | 200 |

| | **Formulae (ppm)** | | | | | | |
|---|---|---|---|---|---|---|---|
| **Ingredient** | **K3** | **L3** | **M3** | **N3** | **03** | **P3** | **Q3** |
| **Log Kill (1 Day)** | | | | | | | |
| S. aureus | 4.4 | 4.3 | 3.0 | 0.3 | 1.3 | 1.7 | 0.1↑ |
| Ps. aeruginosa | ≥4.7 | ≥4.7 | 3.5 | 2.3 | 4.4 | 4.5 | 1.4 |
| E. coli | 0.6 | 0.4 | 0.3 | 0.1 | 0,2 | 0.2 | 0.1 |
| C. albicans | 1.8 | 1.5 | 0.9 | 0.3 | 1.4 | 0.5 | 0.2↑ |
| A. niger | 1.8 | 1.8 | 1.8 | 1.9 | 0.8 | 1.9 | 0.9 |
| **Log Kill (7 Days)** | | | | | | | |
| S. aureus | ≥4.9 | ≥4.9 | ≥4.9 | 3.9 | ≥5.0 | ≥5.0 | ≥5.2 |
| Ps. aeruginosa | ≥4.7 | ≥4.7 | ≥4.7 | ≥4.7 | ≥4.9 | ≥5.0 | ≥5.3 |
| E. coli | ≥5.0 | ≥5.0 | ≥5.0 | 0.6 | 1.9 | 3.8 | ≥5.3 |
| C. albicans | ≥5.0 | ≥5.0 | ≥5.0 | 3.3 | 3.6 | 3.0 | 1.0 |
| A. niger | 1.7 | 1.6 | 1.6 | 1.7 | 1.8 | 1.6 | 1.6 |

| **Log Kill (14 Days)** | | | | | | | |
|---|---|---|---|---|---|---|---|
| S. aureus | ≥4.9 | ≥4.9 | ≥4.9 | ≥4.9 | ≥5.0 | ≥5.0 | NT |
| Ps. aeruginosa | ≥4.7 | ≥4.7 | ≥4.7 | ≥4.7 | ≥4.9 | ≥5.0 | NT |
| E. coli | ≥5.0 | ≥5.0 | ≥5.0 | 0 | 1.3 | ≥5.1 | NT |
| C. albicans | ≥5.0 | ≥5.0 | ≥5.0 | ≥5.0 | 4.7 | 4.0 | NT |
| A. niger | 1.8 | 1.7 | 1.8 | 1.9 | 2.8 | 1.5 | NT |

| **Log Kill (28 Days)** | | | | | | | |
|---|---|---|---|---|---|---|---|
| S. aureus | ≥4.9 | ≥4.9 | ≥4.9 | ≥4.9 | ≥5.0 | ≥5.0 | NT |
| Ps. aeruginosa | ≥4.7 | ≥4.7 | ≥4.7 | ≥4.7 | ≥4.9 | ≥5.0 | NT |
| E. coli | ≥5.0 | ≥5.0 | ≥5.0 | 0 | 2.1 | ≥5.1 | NT |
| C. albicans | ≥5.0 | ≥5.0 | ≥5.0 | ≥5.0 | ≥5.0 | ≥5.0 | NT |
| A. niger | 3.9 | 3.5 | 2.8 | 2.0 | 1.4 | 0.7 | NT |

| | | | | | | | |
|---|---|---|---|---|---|---|---|
| NT = Not Tested | | | | | | | |

Additional compounds were tested alone or in combination. These tests are abbreviated in that they omit tests for fungi. However, experience has shown that combinations meeting the requirements for bacteria virtually always met the fungal requirements as welL Results are given in the following Table 10.

**Table 10.**

| (Borate Saline) | | | | | | | | | | | |
|---|---|---|---|---|---|---|---|---|---|---|---|
| | **Formulae (ppm)** | | | | | | | | | | |
| **Ingredient** | **R3** | **S3** | **T3** | **U3** | **V3** | **W3** | **X3** | **Y3** | **Z3** | **A4** | **B4** |
| Boric Acid | 200 | 200 | 200 | 200 | 200 | 200 | 200 | 200 | 200 | 200 | 200 |
| Sodium Chloride | 800 | 800 | 800 | 800 | 800 | 800 | 800 | 800 | 800 | 800 | 800 |
| Gallic Acid | 100 | 0 | 0 | 0 | 0 | 0 | 0 | 0 | 0 | 0 | 0 |
| Vanillic Acid | 0 | 100 | 200 | 0 | 0 | 0 | 0 | 100 | 0 | 0 | 0 |
| Trans-Cinnamic Acid | 0 | 0 | 0 | 100 | 200 | 500 | 0 | 0 | 0 | 0 | 0 |
| Propyl Gallate | 0 | 0 | 0 | 0 | 0 | 0 | 100 | 0 | 0 | 100 | 0 |
| Oleuropein | 0 | 0 | 0 | 0 | 0 | 0 | 0 | 100 | 100 | 100 | 0 |
| Cinnamaldehyde | 0 | 0 | 0 | 0 | 0 | 0 | 0 | 0 | 0 | 0 | 100 |
| pH | 7.22 | 7.71 | 7.2-7.4 | 7.46 | 7.2-7.4 | 7.2-7.4 | 7.26 | 7.22 | - | 7.45 | 7.42 |
| Color | 0 | 0 | 0 | 0 | 0 | 0 | 0 | 0 | 0 | 0 | 0 |

| **Log Kill (1 Day)** | | | | | | | | | | | |
|---|---|---|---|---|---|---|---|---|---|---|---|
| S. aureus | 1.4 | 0.1 | NT | 0.4 | NT | NT | 0.3 | 0 | 0.3 | 0.1 | NT |
| Ps. aeruginosa | 3.6 | 0.1 | NT | 0.9 | NT | NT | 1.3 | 1.2 | 0.9 | 1.8 | NT |
| E. coli | 0.3 | 0.6 | NT | 0.1 | NT | NT | 0.1 | 0.1 | 0 | 0.1 | NT |

| **Log Kill (7 Days)** | | | | | | | | | | | |
|---|---|---|---|---|---|---|---|---|---|---|---|
| S. aureus | ≥4.6 | 4.6 | ≥4.5 | ≥4.6 | ≥4.5 | ≥4.5 | 1.0 | 2.7 | 2.7 | ≥4.6 | ≥4.9 |
| Ps. aeruginosa | ≥4.7 | .8 | 1.5 | 4.7 | ≥4.6 | 3.5 | 3.3 | 4.4 | 1.1 | ≥4.7 | ≥4.8 |
| E. coli | ≥5.1 | 0.6 | 0.5 | 3.3 | ≥5.2 | 5.2 | 0.5 | 2.7 | 0.5 | ≥5.1 | 4.5 |
| **Cytotoxicity Grade** | | | **0** | | **0** | **0** | | | **0** | **0** | |

| | | | | | | | | | | | |
|---|---|---|---|---|---|---|---|---|---|---|---|
| NT = Not Tested | | | | | | | | | | | |

These results shown that Gallic Acid (R3) meets the criteria for Category 1A PET of USP XXTV and is a useful preservative/disinfectant for ophthalmic solutions and other drug/medical applications. In contrast neither 100 ppm (S3) or 200 ppm (T3) of Vanillic Acid or 100 ppm (Z3) of Oleuropein meet the PET requirements. However, the combination (Y3) of 100 ppm Vanillic Acid with 100 ppm Oleuropein is clearly sunergistic and does meet the Category 1A requirements. Trans-cinnamic Acid meets the PET criteria at either 100 ppm (U3). 200 ppm (V3) or 500 ppm (W3). Significantly the 200 ppm and 500 ppm formulae have a cytotoxicity grade of 0 (USP 26 Biological Reactivity Test) making them especially favorable. Propyl Gallate at 100 ppm does not pass the PET test. However, the combination (A4) of 100 ppm Propyl Gallate with 100 ppm Oleuopein does meet the preservative criteria and has a cytotoxicity grade of 0. Finally 100 ppm of Cinnamaldehyde (B4) passes he preservative tests with excellent results.

The above results demonstrate that a variety of natural products meet the USP preservative criteria for ophthalmic solutions and other pharmaceutical preparations. A recognized standard for evaluating the effectiveness of a preservation agent in contain in the USP. For the reader's convenience the formulae, which meet the USP Category 1A Preservative Effectiveness Test requirements, are summarized in Table 11.

**Table 10.**

| **Ingredient** | **Effective Concentration (ppm)** | **Formula Designators** |
|---|---|---|
| Bilberry Extract | 1000 | J,03 |
| Bilberry Extract | 500 | KK |
| Bilberry Extract | 200 | LL, NN |
| CAPE (Caffeic Acid Phenethyl Esther) | 1000 | P, II |
| CAPE | 700 | EE |
| CAPE | 400 | FF |
| Chlorogenic Acid | 1000 | x |
| Cinnamaldehyde | 100 | B4 |
| Cranberry Extract | 120 | P3 |
| Ferulic Acid | 1000 | W |
| Gallic Acid | 100 | R3 |
| Grape Seed Extract | 1000 | U, A3 |
| Grape Seed Extract | 500 | C3 |
| Grape Seed Extract | 200 | D3 |
| Green Tea Extract | 1000 | R, S, SS |
| Green Tea Extract | 500 | PP |
| Green Tea Extract | 200 | QQ |
| Green Tea Extract | 50 | RR |
| Hydroxytyrosol | 200 | Q3 |
| Oleuropein | 1000 | G3 |
| Oleuropein | 500 | H3 |
| Oleuropein | 200 | I3 |
| Oleuropein | 50 | J3 |
| Pine Bark Extract | 1000 | TT |
| Pomegranate Extract | 1000 | N |
| Pomegranate | 500 | WW |
| Pomegranate | 200 | XX |
| Propyl Gallate + Oleuropein | 100 ppm of each | A4 |
| Pycnogenol | 1000 | UU |
| Quercetin Dihydrate | 1000 | AA |
| Quercetin Dihydrate | 500 | BB |
| Quercetin Dihydrate | 200 | CC |
| Resveratrol | 1000 | K3 |
| Resveratrol | 500 | L3, F3 |
| Resveratrol | 200 | M3 |
| Trans-Cinnamic Acid | 100-500 | U3, V3, W3 |
| Vanillic Acid + Oleuropein | 100 ppm of each | A4 |

Of these perhaps the most promising are Oleuropein (50-200 ppm), Green Tea (200 ppm), Resveratrol (200 ppm), Hydroxytyrosol (200 ppm), Cranberry (120 ppm) and Pomegranate (200 ppm).

Additional combinations of various natural disinfectants were explored. These results are shown in Table 12.

**Table 12.**

| | **Formulae (ppm)** | | | | | | |
|---|---|---|---|---|---|---|---|
| **Ingredients** | **W3** | **X3** | **Y3** | **Z3** | **A4** | **B4** | **C4** |
| Monosodium phosphate | 620 | 620 | 620 | 620 | 620 | 620 | 620 |
| Disodium phosphate | 72 | 72 | 72 | 72 | 72 | 72 | 72 |
| EDTA | 250 | 250 | 250 | 250 | 250 | 250 | 250 |
| Sodium Chloride | 8600 | 8600 | 8600 | 8600 | 8600 | 8600 | 8600 |
| Pluronic F127 | 1000 | 1000 | 1000 | 1000 | 1000 | 1000 | 1000 |
| Oleuropein | 1000 | 0 | 0 | 1000 | 1000 | 0 | 1000 |
| Green Tea | 0 | 500 | 0 | 500 | 0 | 500 | 500 |
| Pomegranate | 0 | 0 | 1000 | 0 | 1000 | 1000 | 1000 |
| | | | | | | | |
| **pH** | 7.6-7.8 | 7.6-7.8 | 7.6-7.8 | 7.6-7.8 | 7.6-7.8 | 7.6-7.8 | 7.6-7.8 |

| **Log Kill (6 hours)** | | | | | | | |
|---|---|---|---|---|---|---|---|
| Ps. aeruginosa | ≥4.9 | ≥4.9 | ≥4.9 | ≥4.9 | ≥4.9 | ≥4.9 | ≥4.9 |
| S. aureus | 0.5 | 1.1 | 2.1 | 1.7 | 1.9 | 1.7 | 1.5 |

| | **Formulae (ppm)** | | | | | | |
|---|---|---|---|---|---|---|---|
| **Ingredients** | **W3** | **X3** | **Y3** | **Z3** | **A4** | **B4** | **C4** |
| Serratia marc. | 1.8 | 2.3 | 2.4 | 2.4 | 3.4 | 3.2 | 2.2 |
| C. albicans | 02 | 1.0 | 1.6 | 1.6 | 1.9 | 2.0 | 2.1 |

These results show that several combinations of the natural antimicrobial agents are synergistic and are extremely effective at a six hour time point. Most of the natural disinfectant antimicrobial agents tested, such as oleuropein, green tea and pomegranate, are extremely effective on *Pseudomonas aeruginosa.* However, the combinations proved to be especially effective against *Serratia marcescens* and *Candida albicans,* The combination of oleuropein and pomegranate or green tea and pomegranate seem particularly effective.

Previous experiments had shown both disinfectant and anti-cytotoxic effects from solutions containing Allantoin. Table 13 shows that there is a positive disinfecting synergy between Allantoin and PHMB.

**Table 13.**

| | **Formulae (ppm)** | |
|---|---|---|
| **Ingredients** | **D4** | **E4** |
| Monosodium phosphate | 62 | 62 |
| Disodium phosphate | 7 | 7 |
| Sodium Chloride | 860 | 860 |
| EDTA | 25 | 25 |
| PHMB | 0.5 | 0.5 |
| Allantoin | 0 | 500 |
| | | |

| **Log Kill (6 hours)** | | |
|---|---|---|
| Ps. aeruginosa | ≥5.1 | ≥5.1 |
| S. aureus | ≥5.1 | 4.3 |
| Serratia marcescens | 1.4 | 3.1 |
| C. albicans | 1.1 | 0.5 |

Allantoin is effective to augment the killing of *Serratia marcesecns* in the presence of PHMB and EDTA. The present inventors reported earlier on the anti-cytotoxic properties of certain natural products. Experiments were under taken to confirm the anti-cytotoxic effects of Allantoin in particular. For this purpose PHMB was also used both because this compound is known to be irritating at effective concentrations and because it was just demonstrated that Allantoin shows synergistic disinfecting properties with PHMB. Besides the usual microbial tests, the material was also used in a cytotoxicity assay, which consisted of cycling high water-content soft contact lenses for five days in each test solution. Then the solution saturated lenses were tested in a L929 cell cytotoxicity model. Cytotoxicity was rated from 0 to 5 with 2 or greater indicating cytotoxicity, Table 14 shows the results of this PHMB/Allantoin experiment

**Table 14.**

| | **Formulae (ppm)** | | | | | | | |
|---|---|---|---|---|---|---|---|---|
| **Ingredients** | **G4** | **H4** | **14** | **J4** | **K4** | **M4** | **N4** | **04** |
| Monosodium phosphate | 100 | 100 | 100 | 100 | 100 | 100 | 100 | 100 |
| Disodium phosphate | 400 | 400 | 400 | 400 | 400 | 400 | 400 | 400 |
| Sodium Chloride | 700 | 700 | 700 | 700 | 700 | 700 | 700 | 700 |
| EDTA | 0 | 0 | 50 | 0 | 0 | 0 | 0 | 0 |
| PHMB | 2 | 1 | 1 | 2 | 1 | 3 | 2 | 3 |
| Allantoin | 50 | 50 | 50 | 100 | 100 | 50 | 0 | 0 |
| | | | | | | | | |

| **Log Kill (6 hours)** | | | | | | | | |
|---|---|---|---|---|---|---|---|---|
| S. aureus | ≥4.8 | 4.5 | ≥4.8 | ≥4.8 | 4.5 | ≥4.8 | ≥4.8 | ≥4.8 |
| Ps. aeruginosa | ≥5.0 | ≥5.0 | ≥5.0 | ≥5.0 | ≥5.0 | ≥5.0 | ≥5.0 | ≥5.0 |
| C. albicans | 1.7 | 1.4 | 0.6 | 1.3 | 1.0 | 3.3 | 2.2 | 3.6 |
| | | | | | | | | |
| **Cytotoxic Response** | 0 | 0 | 0 | 0 | 0 | 0 | 2+ | 2+ |

The PHMB is extremely effective against the tested bacteria, but except at the highest concentrations it is only moderately effective against *Candida albicans.* Unfortunately, the higher concentrations of PHMB show a significant level of cytotoxicity. Comparison should be made between formulations M4 and 04. Whereas 04 shows significant cytotoxicity, M4 shows no cytotoxicity despite the fact that the two formulae are essentially identical in disinfecting ability. The difference between the formulae is the presence of 500 ppm Allantoin in M4. This indicates that relatively low concentrations of Allantoin can mitigate the cytotoxic characteristics of PHMB without impairing the disinfecting power of PHMB. Thus at lower PHMB concentrations addition of Allantoin can augment antimicrobial activity (Table 13), and at higher PHMB concentration the addition of Allantoin suppresses the cytotoxic effects of the PHMB (Table 14).

Cytotoxicity studies were also performed to determine the potential of these natural compounds, extracts and derivatives for irritation and discomfort when used as disinfecting or preservative agents.

The cytotoxicity test analysis was performed in accordance with USP 24 <87> Biological Reactivity Tests, In Vitro, and USP Elution. Two ml of each sample were diluted with 10 ml of 1 x MEM. The biological reactivity were described and rated as 0 (no reactivity), 1 (slight reactivity), 2 (mild reactivity), 3 (moderate reactivity), 4 (severe reactivity). The test results are presented in Table 15 for Oleuropein, Resveratrol, Pomegranate, Green Tea, Cranberry and Hydroxytyrosol.

**Table 15**

| Compound/Extract | Concentration | Grade | Reactivity |
|---|---|---|---|
| Oleuropein | 50 ppm | 0 | None |
| Oleuropein | 200 ppm | 0 | None |
| Oleuropein | 1000 ppm | 0 | None |
| Green Tea | 50 ppm | 0 | None |
| Green Tea | 200 | 0 | None |
| Green Tea | 1000 ppm | 0 | None |
| Resveratrol | 50 ppm | 0 | None |
| Resveratrol | 200 ppm | 0 | None |
| Pomegranate | 50 ppm | 0 | None |
| Pomegranate | 200 ppm | 0 | None |
| Cranberry | 50 ppm | 0 | None |
| Cranberry | 200 ppm | 0 | None |
| Hydroxytyrosol | 200 ppm | 0 | None |
| BAK | 100 ppm | 4 | Severe |
| Test Vehicle | Borate Buffered Saline | 0 | None |
| (-) Control | As per the USP | 0 | None |
| (+) Control | As per the USP | 4 | Severe |

The remarkable result of this series of experiments is that the natural compounds, extracts and derivatives all showed essentially no biological reactivity in this very sensitive cytotoxicity test regimen. Compared to a typically used concentration of a chemical agent, BAK in a similar vehicle, the natural ingredients far outperformed the chemical agent in the degree of cytotoxicity demonstrated. Combined with the antimicrobial results previously presented, the natural compounds, extracts and derivatives present an unexpected degree of antimicrobial efficacy (preservation and disinfection) and lack of cytotoxicity.

The following claims are thus to be understood to include what is specifically illustrated and described above, what is conceptually equivalent, what can be obviously substituted and also what incorporates the essential idea of the invention. Those skilled in the art will appreciate that various adaptations and modifications of the just-described preferred embodiment can be configured without departing from the scope of the invention. The illustrated embodiment has been set forth only for the purposes of example and that should not be taken as limiting the invention. Therefore, it is to be understood that, within the scope of the appended claims, the invention may be practiced other than as specifically described herein.

### Clauses - The following clauses define features of the invention.

Clause 1. A preparation for topical application containing between 10 and 10,000 parts per million of a naturally-occurring antimicrobial agent selected from the group consisting of oleuropein, gallic acid, cinnamaldehyde, green tea extract, resveratrol, trans-cinnamic acid, pomegranate extract, hydroxytyrosol and cranberry extract, oleuropein, green tea extract, hydroxytyrosol, and pomegranate extract.
Clause 2. The preparation according to Clause 1 formulated for ophthalmic application.
Clause 3. The preparation according to Clause 2 further comprising a physiologically compatible buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 4. The preparation according to Clause I formulated for epidermal application.
Clause 5. The preparation according to Clause 1 formulated for application to mucus membranes.
Clause 6. The preparation according to Clause 1, wherein the naturally-occurring antimicrobial agent is necessary for antimicrobial preservation of the preparation.
Clause 7. The preparation according to Clause 1, wherein the naturally-occurring antimicrobial agent is solely responsible for antimicrobial preservation of the preparation.
Clause 8. A pharmaceutical preparation preserved with between 10 and 10,000 parts per million of a naturally occurring antimicrobial agent selected from the group consisting of oleuropein, gallic acid, cinnamaldehyde, green tea extract, resveratrol, trans-cinnamic acid, pomegranate extract, hydroxytyrosol and cranberry extract, oleuropein, green tea extract, hydroxytyrosol, and pomegranate extract.
Clause 9. A pharmaceutical or topical preparation containing between 10 and 5,000 parts per million of an antimicrobial agent selected from the group consisting of gallic acid, trans-cinnamic acid and cinnamaldehyde.
Clause 10. The preparation according to Clause 9 formulated for ophthalmic application.
Clause 11. The preparation according to Clause 10 further comprising a buffer selected from the group consisting of phosphate, bicarbonate, citrate, borate, ACES, BBS, BICINE, BIS-Tris, BIS-Tris Propane, HEPES, HBPPS, imidazole, MES, MOPS, PIPES, TAPS, TES, and Tricinc.
Clause 12. The preparation according to Clause 9 formulated for epidermal application.
Clause 13. The preparation according to Clause 9 formulated for application to mucus membranes.
Clause 14. The preparation according to Clause 9, wherein the naturally occurring antimicrobial agent is necessary for antimicrobial preservation of the preparation.
Clause 15. The preparation according to Clause 9, wherein the naturally occurring antimicrobial agent is solely responsible for antimicrobial preservation of the preparation.
Clause 16. A pharmaceutical or topical preparation containing between 100 and 5,000 parts per million of caffeic acid phenyl ester as an antimicrobial agent.
Clause 17. The preparation according to Clause 16 formulated for ophthalmic application.
Clause 18. The preparation according to Clause 17, further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 19. The preparation according to Clause 16 formulated for epidermal application.
Clause 20. The preparation according to Clause 16 formulated for application to mucus membranes.
Clause 21. The preparation according to Clause 16, wherein the caffeic acid phenyl ester is necessary for antimicrobial preservation of the preparation.
Clause 22. The preparation according to Clause 16, wherein the caffeic acid phenyl ester is solely responsible for preservation of the preparation.
Clause 23. A pharmaceutical or topical preparation containing between 10 and 10,000 parts per million of oleuropein as an antimicrobial agent.
Clause 24. The preparation according to Clause 23 formulated for ophthalmic application.
Clause 25. The preparation according to Clause 24 further comprising a buffer selected from the group consisting of phosphate, bicarbonate, citrate, borate, ACES, BES, BIC]NE, BIS-Tris, BIS-Tris Propane, HEPES, HEPPS, imidazole, MES, MOPS, PIPES, TAPS, TES, and Tricine.
Clause 26. The preparation according to Clause 23 formulated for epidermal application.
Clause 27. The preparation according to Clause 23 formulated for application to mucus membranes.
Clause 28. The preparation according to Clause 23, wherein the oleuropein is necessary for antimicrobial preservation of the preparation.
Clause 29. The preparation according to Clause 23, wherein the oleuropein is solely responsible for antimicrobial preservation of the preparation.
Clause 30. A pharmaceutical or topical preparation containing between 10 and 1,000 parts per million of cranberry extract as an antimicrobial agent.
Clause 31. The preparation according to Clause 30 formulated for ophthalmic application.
Clause 32. The preparation according to Clause 31 further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricinc.
Clause 33. The preparation according to Clause 30 formulated for epidermal application.
Clause 34. The preparation according to Clause 30 formulated for application to mucus membranes.
Clause 35. The preparation according to Clause 30, wherein the cranberry extract is necessary for antimicrobial preservation of the preparation.
Clause 36. The preparation according to Clause 30, wherein the cranberry extract is solely responsible for antimicrobial preservation of the preparation.
Clause 37. A pharmaceutical or topical preparation containing between 100 and 5,000 parts per million of grape seed extract as an antimicrobial agent.
Clause 38. The preparation according to Clause 37 formulated for ophthalmic application.
Clause 39. The preparation according to Clause 38 further comprising a buffer selected from the group consisting of phosphate, bicarbonate, citrate, borate, ACES, BES, BICINE, BIS-Tris, BIS-Tris Propane, HEPES, IIEPPS, imidazole, MES, MOPS, PIPES, TAPS, TES, and Tricine.
Clause 40. The preparation according to Clause 37 formulated for epidermal application.
Clause 41. The preparation according to Clause 37 formulated for application to mucus membranes.
Clause 42. The preparation according to Clause 37, wherein the grape seed extract is necessary for antimicrobial preservation of the preparation.
Clause 43. The preparation according to Clause 37, wherein the grape seed extract is solely responsible for antimicrobial preservation of the preparation.
Clause 44. A pharmaceutical or topical preparation containing between 10 and 5,000 parts per million of green tea extract as an antimicrobial agent.
Clause 45. The preparation according to Clause 44 formulated for ophthalmic application.
Clause 46. The preparation according to Clause 45 further comprising a buffer selected from the group consisting of phosphate, bicarbonate, citrate, borate, ACES, BBS, BICINE, BIS-Tris, BIS-Tris Propane, HEPES, HEPPS, imidazole, MES, MOPS, PIPES, TAPS, TES, and Tricine.
Clause 47. The preparation according to Clause 44 formulated for epidermal application.
Clause 48. The preparation according to Clause 44 formulated for application to mucus membranes.
Clause 49. The preparation according to Clause 44, wherein the green tea extract is necessary for antimicrobial preservation of the preparation.
Clause 50. The preparation according to Clause 44, wherein the green tea extract is solely responsible for antimicrobial preservation of the preparation.
Clause 51. A preparation for topical application containing between 10 and 1,000 parts per million of hydroxytyrosol as an antimicrobial agent.
Clause 52. The preparation according to Clause 51 formulated for ophthalmic application.
Clause 53. The preparation according to Clause 52 further comprising a physiologically compatible buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 54. The preparation according to Clause 51 formulated for epidermal application.
Clause 55. The preparation according to Clause 51 formulated for application to mucus membranes.
Clause 56. The preparation according to Clause 51, wherein the hydroxytyrosol is necessary for antimicrobial preservation of the preparation.
Clause 57. The preparation according to Clause 51, wherein the hydroxytyrosol is solely responsible for antimicrobial preservation of the preparation.
Clause 58. A pharmaceutical or topical preparation containing between 10 and 5,000 parts per million of pine bark extract as an antimicrobial agent.
Clause 59. The preparation according to Clause 58, wherein the pine bark extract is pycnogenol.
Clause 60. The preparation according to Clause 58 formulated for ophthalmic application.
Clause 61. The preparation according to Clause 60 further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 62. The preparation according to Clause 58 formulated for epidermal application.
Clause 63. The preparation according to Clause 58 formulated for application to mucus membranes.
Clause 64. The preparation according to Claim 58, wherein the pine bark extract is necessary for antimicrobial preservation of the preparation.
Clause 65. The preparation according to Clause 58, wherein the pine bark extract is solely responsible for antimicrobial preservation of the preparation.
Clause 66. A pharmaceutical or topical preparation containing between 10 and 5,000 parts per million of pomegranate extract as an antimicrobial agent.
Clause 67. The preparation according to Clause 66 formulated for ophthalmic application.
Clause 68. The preparation according to Clause 67 further comprising a physiologically compatible buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 69. The preparation according to Clause 66 formulated for epidermal application.
Clause 70. The preparation according to Clause 66 formulated for application to mucus membranes.
Clause 71. The preparation according to Clause 66, wherein the pomegranate extract is necessary for antimicrobial preservation of the preparation.
Clause 72. The preparation according to Clause 66, wherein the pomegranate extract is solely responsible for antimicrobial preservation of the preparation.
Clause 73. A pharmaceutical or topical preparation containing between 100 and 5,000 parts per million of resveratrol as an antimicrobial agent.
Clause 74. The preparation according to Clause 73 formulated for ophthalmic application.
Clause 75. The preparation according to Clause 73, further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 76. The preparation according to Clause 73 formulated for epidermal application.
Clause 77. The preparation according to Clause 73 formulated for application to mucus membranes.
Clause 78. The preparation according to Clause 73, wherein the resveratrol is necessary for antimicrobial preservation of the preparation.
Clause 79. The preparation according to Clause 73, wherein the resveratrol is solely responsible for antimicrobial preservation of the preparation.
Clause 80. A pharmaceutical or topical preparation preserved by a combination of between 10 and 5,000 parts per million of oleuropein with between 10 and 5,000 parts per million green tea extract as antimicrobial agents.
Clause 81. The preparation according to Clause 80 formulated for ophthalmic application.
Clause 82. The preparation according to Clause 81 further comprising a buffer selected from the group consisting of phosphate, bicarbonate, citrate, borate, ACES, BES, BICINE, BIS-Tris, BIS-Tris Propane, HEPES, HBPPS, imidazole, MES, MOPS, PIPES, TAPS, TES, and Tricine.
Clause 83. The preparation according to Clause 80 formulated for epidermal application.
Clause 84. The preparation according to Clause 80 formulated for application to mucus membranes.
Clause 85. The preparation according to Clause 80, wherein the oleuropein and the green tea extract are necessary for antimicrobial preservation of the preparation.
Clause 86. The preparation according to Clause 80, wherein the oleuropein and the green tea extract are solely responsible for antimicrobial preservation of the preparation.
Clause 87. A pharmaceutical or topical preparation preserved by a combination of between 10 and 5,000 parts per million of oleuropein with between 10 and 5,000 parts per million pomegranate extract as antimicrobial agents.
Clause 88. The preparation according to Clause 87 formulated for ophthalmic application.
Clause 89. The preparation according to Clause 88 further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 90. The preparation according to Clause 87 formulated for epidermal application.
Clause 91. The preparation according to Clause 87 formulated for application to mucus membranes.
Clause 92. The preparation according to Clause 87, wherein the oleuropein and the pomegranate extract are necessary for antimicrobial preservation of the preparation.
Clause 93. The preparation according to Clause 87, wherein the oleuropein and the pomegranate extract are solely responsible for antimicrobial preservation of the preparation.
Clause 94. A pharmaceutical or topical preparation preserved by a combination of between 10 and 5,000 parts per million of pomegranate extract with between 10 and 5,000 parts per million green tea extract as antimicrobial agents.
Clause 95. The preparation according to Clause 94 formulated for ophthalmic application.
Clause 96. The preparation according to Clause 95 further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 97. The preparation according to Clause 94 formulated for epidermal application.
Clause 98. The preparation according to Clause 94 formulated for application to mucus membranes.
Clause 99. The preparation according to Clause 94, wherein the green tea extract and the pomegranate extract are necessary for antimicrobial preservation of the preparation.
Clause 100. The preparation according to Clause 94, wherein the green tea extract and the pomegranate extract are solely responsible for antimicrobial preservation of the preparation.
Clause 101. A pharmaceutical or topical preparation preserved by a combination of between 10 and 500 parts per million of vanillic acid with between 10 and 200 parts per million oleuropein as antimicrobial agents.
Clause 102. The preparation according to Clause 101 formulated for ophthalmic application.
Clause 103. The preparation according to Clause 101 further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 104. The preparation according to Clause 101 formulated for epidermal application.
Clause 105. The preparation according to Clause 101 formulated for application to mucus membranes.
Clause 106. The preparation according to Clause 101, wherein the vanillic acid and the oleuropein are necessary for antimicrobial preservation of the preparation.
Clause 107. The preparation according to Clause 101, wherein the vanillic acid and the olcuropein are solely responsible for antimicrobial preservation of the preparation.
Clause 108. A pharmaceutical or topical preparation preserved by a combination of between 10 and 500 parts per million of propyl gallate with between 10 and 200 parts per million oleuropein as antimicrobial agents.
Clause 109. The preparation according to Clause 108 formulated for ophthalmic application.
Clause 110. The preparation according to Clause 108 further comprising a buffer selected from the group consisting of phosphate, bicarbonate, citrate, borate, ACES, BBS, BICINE, BIS-Tris, BIS-Tris Propane, HEPES, HEPPS, imidazole, MES, MOPS, PIPES, TAPS, TES, and Tricine.
Clause 111. The preparation according to Clause 108 formulated for epidermal application.
Clause 112. The preparation according to Clause 108 formulated for application to mucus membranes.
Clause 113. The preparation according to Clause 108, wherein the propyl gallate and the oleuropein are necessary for antimicrobial preservation of the preparation.
Clause 114. The preparation according to Clause 108, wherein the propyl gallate and the oleuropein are solely responsible for antimicrobial preservation of the preparation.
Clause 115. A pharmaceutical or topical preparation preserved by between 10 and 5,000 parts per million gallic acid as a antimicrobial agent.
Clause 116. The preparation according to Clause 115 formulated for ophthalmic application.
Clause 117. The preparation according to Clause 115 further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 118. The preparation according to Clause 115 formulated for epidermal application.
Clause 119. The preparation according to Clause 115 formulated for application to mucus membranes.
Clause 120. The preparation according to Clause 115, wherein the gallic acid is necessary for antimicrobial preservation of the preparation.
Clause 121. The preparation according to Clause 115, wherein the gallic acid is solely responsible for antimicrobial preservation of the preparation.
Clause 122. A pharmaceutical or topical preparation preserved by between 10 and 5,000 parts per million trans-cinnamic acid as a antimicrobial agent.
Clause 123. The preparation according to Clause 122 formulated for ophthalmic application.
Clause 124. The preparation according to Clause 122 further comprising a buffer selected from the group consisting of phosphate, bicarbonate, citrate, borate, ACES, BES, BICINE, BIS-Tris, BIS-Tris Propane, IIEPES, HEPPS, imidazole, MES, MOPS, PIPES, TAPS, TES, and Tricine.
Clause 125. The preparation according to Clause 122 formulated for epidermal application.
Clause 126. The preparation according to Clause 122 formulated for application to mucus membranes.
Clause 127. The preparation according to Clause 122, wherein the gallic acid is necessary for antimicrobial preservation of the preparation.
Clause 128. The preparation according to Clause 122, wherein the gallic acid is solely responsible for antimicrobial preservation of the preparation.
Clause 129. A pharmaceutical or topical preparation preserved by between 10 and 5,000 parts per million cinnamaldehyde as a antimicrobial agent.
Clause 130. The preparation according to Clause 129 formulated for ophthalmic application.
Clause 131. The preparation according to Clause 129 further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 132. The preparation according to Clause 129 formulated for epidermal application.
Clause 133. The preparation according to Clause 129 formulated for application to mucus membranes.
Clause 134. The preparation according to Clause 129, wherein the cinnamaldehyde is necessary for antimicrobial preservation of the preparation.
Clause 135. The preparation according to Clause 129, wherein the cinnamaldehyde is solely responsible for antimicrobial preservation of the preparation.
Clause 136. A preparation for topical application containing a combination of between 1 and 5 parts per million of polyhexamethyl biguanidine with between 10 and 1,000 parts per million allantoin as antimicrobial agents.
Clause 137. The preparation according to Clause 136 formulated for ophthalmic application.
Clause 138. The preparation according to Clause 137 further comprising a buffer selected from the group consisting of phosphate, bicarbonate, citrate, borate, ACES, BES, BICINE, BIS-Tris, BIS-Tris Propane, HEPES, HEPPS, imidazole, MES, MOPS, PIPES, TAPS, TES, and Tricine.
Clause 139. The preparation according to Clause 136 formulated for epidermal application.
Clause 140. The preparation according to Clause 136 formulated for application to mucus membranes.
Clause 141. The preparation according to Clause 136, wherein the polyhexamethyl biguanidine and the allantoin are necessary for antimicrobial preservation of the preparation.
Clause 142. The preparation according to Clause 136, wherein the polyhexamethyl biguanidine and the allantoin arc solely responsible for antimicrobial preservation of the preparation.
Clause 143. A method of reducing cytotoxicity of topical preparations containing irritating chemical preservative agents comprising the step of adding between 10 and 1,000 parts per million allantoin.
Clause 144. The method according to Clause 143, wherein the irritating chemical preservative is a biguanidine preservative.

## Claims

1. A preparation for topical application containing between 10 and 10,000 parts per million of green tea extract as an antimicrobial agent.

2. The preparation according to Claim 1, containing between 10 and 5,000 parts per million of the green tea extract as the antimicrobial agent.

3. The preparation according to any of the preceding claims, wherein the green tea extract is necessary for antimicrobial preservation of the preparation.

4. The preparation according to Claims 1 or 2, wherein the green tea extract is solely responsible for antimicrobial preservation of the preparation.

5. The preparation according to any one of Claims 1 to 3, further comprising between 10 and 10,000 parts per million of oleuropein as an additional antimicrobial agent.

6. The preparation according to Claim 5, wherein the oleuropein and the green tea extract are necessary for antimicrobial preservation of the preparation.

7. The preparation according to Claim 5, wherein the oleuropein and the green tea extract are solely responsible for antimicrobial preservation of the preparation.

8. The preparation according to any one of Claims 1 to 3, further comprising between 10 and 10,000 parts per million of pomegranate extract as an additional antimicrobial agent.

9. The preparation according to Claim 8, wherein the pomegranate extract and the green tea extract are necessary for antimicrobial preservation of the preparation.

10. The preparation to Claim 8, wherein the pomegranate extract and the green tea extract are solely responsible for antimicrobial preservation of the preparation.

11. A preparation according to any of Claims 1 to 3, further comprising between 10 and 10,000 parts per million of oleuropein as an additional antimicrobial agent and further comprising between 10 and 10,000 parts per million of pomegranate extract as an additional antimicrobial agent.

12. The preparation according to any of the preceding claims, further comprising a physiologically compatible buffer selected from the group consisting of phosphate, bicarbonate, citrate, borate, ACES, BES, BIGINE, BIS-Tris, BIS-Tris Propane, HEPES, HEPPS, imidazole, MES, MOPS, PIPES, TAPS, TES, and Tricine.

13. The preparation according to any of the preceding claims, formulated for ophthalmic application.

14. The preparation according to any of the preceding claims, formulated for epidermal application.

15. The preparation according to any of the preceding claims, formulated for application to mucus membranes.
